# EUROPÄISCHE PATENTSCHRIFT

(11) **EP 0 720 840 B1**
(45) Veröffentlichungstag und Bekanntmachung des Hinweises auf die Patenterteilung: **11.03.1998**
(21) Anmeldenummer: 95119740.9
(22) Anmeldetag: 14.12.1995
(51) Int. Cl.: A61F 2/44

(54) **Platzhalter für Wirbel**
Space keeping device for a vertebra
Dispositif pour garder un espace pour une vertèbre

(30) Priorität: 04.01.1995 DE 19500170
(43) Veröffentlichungstag der Anmeldung: 10.07.1996
(73) Patentinhaber: BIEDERMANN MOTECH GmbH, D-78054 Villingen-Schwenningen (DE)
(72) Erfinder: Biedermann, Lutz, D-78048 VS-Villingen (DE); Harms, Jürgen, Prof. Dr., D-76337 Waldbronn (DE)
(74) Vertreter: Prüfer, Lutz H., Dipl.-Phys.

(56) Entgegenhaltungen:
- EP-A- 0 268 115
- WO-A-94/18913
- US-A- 3 426 364

## Beschreibung

Die Erfindung betrifft einen Platzhalter für Wirbel.

In einem Fall, in dem ein oder mehrere Wirbel aus der Wirbelsäule entfert werden müssen, ist es erforderlich, einen Platzhalter zwischen die verbleibenden Teile der Wirbelsäule einzusetzen.

Aus der EP 0 268 115 B1 ist ein Platzhalter insbesondere für einen Wirbel bekannt, der ein mantelförmiges Element mit wenigstens einer Ausnehmung in der Wandung aufweist, wobei der obere und untere Rand des mantelförmigen Elements jeweils wenigstens teilweise zackenförmig ausgebildet ist. Das mantelförmige Element weist über seine gesamte axiale Länge einen gleichbleibenden Querschnitt auf.

Für den Fall, daß zwei sehr unterschiedliche Querschnitte aufweisende Wirbel der Wirbelsäule miteinander verbunden werden sollen, ist jedoch ein Platzhalter der oben beschriebenen Art ungünstig, da der Querschnitt des Platzhalters entweder zu groß für die Verbindung mit dem kleineren Wirbel oder zu klein für die Verbindung mit dem größeren Wirbel ist. Dadurch ist eine optimale kraftschlüssige Verbindung nicht gewährleistet.

Aus der WO-A-9 418 913 ist ein Platzhalter für Wirbel nach dem Oberbegriff des Patentanspruches 1 bekannt.

Aufgabe der Erfindung ist es, einen Platzhalter für Wirbel zu schaffen, der so ausgebildet ist, daß er zur Verbindung zweier Wirbel oder zweier Knochenstücke unterschiedlicher Größe und unterschiedlichen Querschnittes oder zur platzsparenden Verbindung zweier Wirbel gleicher Größe geeignet ist.

Die Aufgabe wird gelöst durch einen Platzhalter für Wirbel nach dem Patentanspruch 1. Weiterbildungen sind in den Unteransprüchen gegeben.

Der Platzhalter für Wirbel hat den Vorteil, daß eine sehr einfache Anpassung an die Größe der zu verbindenden Wirbel und ein sehr einfacher Ersatz für Wirbel der geeigneten Größe zwischen die zu verbindenden Wirbel gegeben ist.

Weitere Merkmale und Zweckmäßigkeiten der Erfindung ergeben sich aus der Beschreibung von Ausführungsbeispielen anhand der Figuren.

Von den Figuren zeigen:
- Fig. 1:: eine teilgeschnittene Vorderansicht eines ersten Ausführungsbeispiels eines Platzhalters für Wirbel gemäß der Erfindung;
- Fig. 2:: eine teilgeschnittene Vorderansicht eines zweiten Ausführungsbeispiels eines Platzhalters für Wirbel gemäß der Erfindung;
- Fig. 3a) bis 3c):: teilgeschnittene Vorderansichten von Verbindungsstücken für Platzhalter gemäß einem Ausführungsbeispiel der Erfindung.

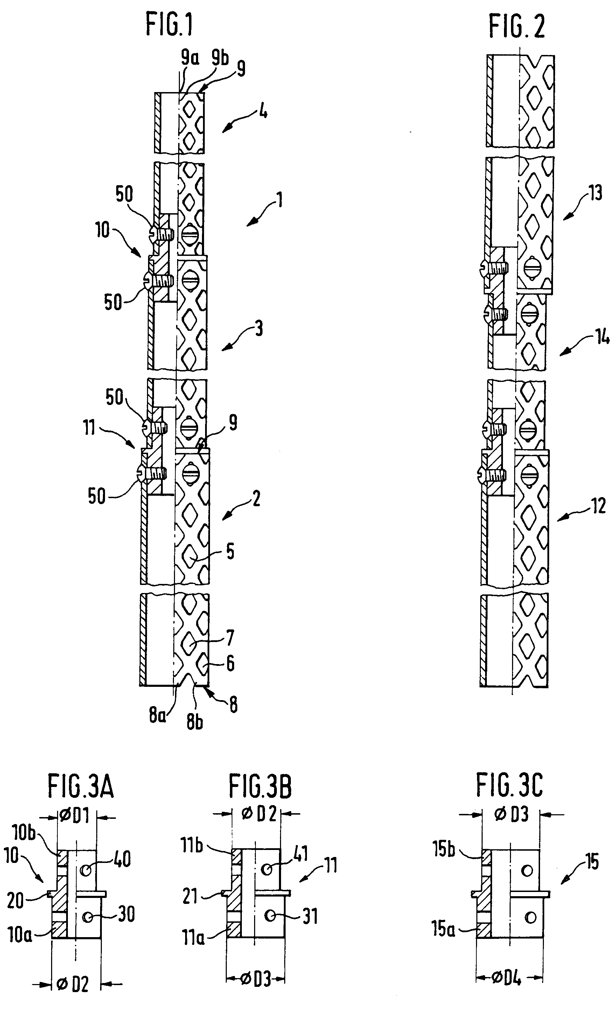

Wie aus Fig. 1 ersichtlich ist, ist der Platzhalter 1 aus drei zylindermantelförmigen Elementen 2, 3, 4 gebildet, die über Verbindungsstücke 10, 11 miteinander verbunden sind. Der Zylindermantel eines jeden der zylindermantelförmigen Elemente 2, 3, 4 weist in der aus Fig. 1 ersichtlichen Weise rautenförmige Ausnehmungen 5 auf, die sich mit ihrer Längsdiagonalen parallel zu der Zylinderachse erstrecken. Jeweils benachbarte Reihen 6, 7 solcher rautenförmigen Ausnehmungen sind gegeneinander um eine halbe Rautenhöhe versetzt. Durch die derart netzförmig ausgebildete Wandung der zylindermantelförmigen Elemente (2,3,4) wird erreicht, daß eine auf die Elemente in Richtung ihrer Längsachse wirkende Belastung gleichmäßig aufgenommen wird.

Der untere Rand 8 und der obere Rand 9 jedes zylindermantelförmigen Elementes ist jeweils so ausgebildet, daß annähernd "V"-förmige Zacken 8a, 8b bzw. 9a, 9b in der Mantelebene jeweils parallel zur Zylinderachse nach unten bzw. nach oben hervorstehen. Die Enden der Zacken 8a, 8b bzw. 9a, 9b sind so angefast bzw. angeschrägt, daß sich die beiden schrägen Flächen unter einem Winkel von annähernd 45° schneiden, so daß eine Art Schneidrand gebildet ist.

Bei dem in Fig. 1 dargestellten Ausführungsbeispiel umfaßt der Platzhalter 1 drei zylindermantelförmige Elemente 2, 3, 4 der oben beschriebenen Art, wobei die Elemente jeweils gleiche Wandstärke aber unterschiedliche Mantelquerschnittsflächen aufweisen. Ein Innendurchmesser D2 des zylindermantelförmigen Elementes 3 ist kleiner als ein Innendurchmesser D3 des zylindermantelförmigen Elementes 2 und ein Innendurchmesser D1 des zylindermantelförmigen Elementes 4 ist kleiner als der Innendurchmesser D2 des zylindermantelförmigen Elementes 3. Die Elemente 2 und 3 bzw. 4 und 3 sind jeweils über Verbindungsstücke 10 bzw. 11 miteinander verbunden.

Wie aus Fig. 3a und Fig. 3b ersichtlich ist, bestehen die Verbindungsstücke 10 bzw. 11 jeweils aus einem ersten zylindermantelförmigen Abschnitt 10a bzw. 11a und einem zweiten zylindermantelförmigen Abschnitt 10b bzw. 11b. Ein Außendurchmesser D3 des Abschnittes 11a des Verbindungsstückes 11 entspricht dem Innendurchmesser des Elementes 2. Ein Außendurchmesser D2 des Abschittes 11b des Verbindungsstückes 11 entspricht dem Innendurchmesser des Elementes 3. Ein Außendurchmesser D2 des Abschnittes 10a des Verbindungsstückes 10 entspricht dem Innendurchmesser des Elementes 3. Ein Außendurchmesser D1 des Abschnittes 10b des Verbindungsstückes 10 entspricht dem Innendurchmesser des Elementes 4.

Die ersten Abschnitte 10a bzw. 11a der Verbindungselemente 10 bzw. 11 weisen jeweils an ihrer an den zweiten Abschnitt 10b bzw. 11b angrenzenden Seite einen Vorsprung 20 bzw. 21 auf, dessen Außendurchmesser einem Außendurchmesser des Elementes 3 bzw. des Elementes 2 entspricht.

Der erste zylindermantelförmigen Abschnitt 10a bzw. 11a sowie der zweite zylindermantelförmigen Abschnitt 10b bzw. 11b der Verbindungsstücke 10 bzw. 11 weist jeweils eine Gewindebohrung 30, 40 bzw. 31, 41 zur Aufnahme einer Schraube 50 auf, deren Achse in einem rechten Winkel zur Zylinderachse verläuft. Der Durchmesser der Gewindebohrungen 30, 40, 31, 41 ist kleiner als der Abstand zweier gegenüberliegender Seiten der rautenförmigen Ausnehmung 5 der zylindermantelförmigen Elemente 2, 3 bzw. 4.

Der Abstand der Achse der Gewindebohrungen 30, 40 bzw. 31, 41 der Verbindungsstücke 10 bzw. 11 von den jeweiligen Vorsprüngen 20 bzw. 21 ist gleich dem Abstand eines Mittelpunktes einer ersten vollständigen rautenförmigen Ausnehmung 5 von dem Rand 8 bzw. 9 des zylinderförmigen Elementes 2, 3 bzw. 4. In zusammengesetztem Zustand sind die Elemente 2, 3, 4 des Platzhalters mit den Verbindungsstücken 10 bzw. 11 derart verschraubt, daß jeweils eine durch eine der ersten vollständigen Ausnehmungen 5 hindurchgeführte Schraube 50 in die jeweilige Gewindebohrung des Verbindungsstückes eingreift. Dabei weist ein Schraubenkopf der Schraube einen Durchmesser auf, der größer als der Abstand zweier gegenüberliegender Seiten der rautenförmigen Ausnehmung 5 ist.

Die zylindermantelförmigen Elemente 2, 3 und 4, die Verbindungsstücke 10, 11 sowie die Schrauben 50 sind aus körperverträglichem Material, beispielsweise aus Titan gefertigt.

Im Betrieb werden die Querschnitte bzw. die Durchmesser der zylindermantelförmigen Elemente 2 und 4 so ausgewählt, daß sie im wesentlichen den Querschnitten bzw. den Durchmessern der miteinander zu verbindenen Wirbeln entsprechen. Der Querschnitt bzw. der Durchmesser des zylindermantelförmigen Elementes 3 wird so gewählt, daß er kleiner als der Durchmesser des zylindermantelförmigen Elementes 2 und größer als der Durchmesser des zylindermantelförmigen Elementes 4 ist. Die axialen Längen der zylindermantelförmigen Elemente 2, 3 und 4 werden so gewählt, daß in zusammengestecktem Zustand der Platzhalter 1 so zwischen die zu verbindenden Wirbel paßt, daß diese den ursprünglichen Abstand zueinander behalten. Anschließend werden die zylindermantelförmigen Elemente 4 bzw. 3 jeweils so über den ersten Abschnitt 10a bzw. den zweiten Abschnitt 10b des Verbindungsstückes 10 geschoben, daß die einander zugewandten Enden der Elemente 4 und 3 jeweils an dem Vorsprung 20 des Verbindungsstückes 10 anliegen. Dann wird jeweils eine der rautenförmigen Ausnehmungen 5 der Wandung der Elemente 2 bzw. 3 durch Drehen des Elementes in Deckung mit der Gewindebohrung 30 bzw. 40 des Verbindungsstückes 10 gebracht. Wie aus Fig. 1 ersichtlich ist, werden die Elemente 4 und 3 sodann jeweils über die Schrauben 50, die in die Gewindebohrungen 30 bzw. 40 eingeschraubt werden, mit dem Verbindungsstück 10 verbunden. In analoger Weise werden die Elemente 2 und 3 mit dem Verbindungsstück 11 verbunden.

Sodann greift der Platzhalter 1 mit seinen Zacken 8a, 8b des Elementes 2 bzw. mit den Zacken 9a, 9b des Elementes 4 in die Stirnseite der zu verbindenden Wirbel in der Weise ein, daß eine Torsionsbewegung des einen Wirbels gegenüber dem anderen Wirbel durch die hervorstehenden Zacken auf den jeweils anderen Wirbel übertragen bzw. gebremst wird.

Durch die Ausnehmungen hindurch wird zumindest das Innere des Platzhalters 1 mit Knochenzement ausgefüllt. Gewünschtenfalls kann so viel Knochenzement eingeführt werden, daß dieser durch die Ausnehmungen nach außen tritt und daß eine Modellierung auf der Außenfläche vorgenommen wird. Vorzugsweise wird das Ausfüllen mit Knochenzement nach dem Einsetzen des Platzhalters 1 vorgenommen. Es ist aber auch ein Ausfüllen des Innenraumes des Platzhalters 1 vor dem Einsetzen möglich.

Bei dem in Fig. 2 dargestellten Platzhalter gemäß eines zweiten Ausführungsbeispiels ist zwischen zwei zylindermantelförmige Elementen 12, 13 mit gleichem Durchmesser ein drittes zylindermantelförmiges Element 14 mit kleinerem Durchmesser vorgesehen. Die Elemente 12, 13 und 14 weisen jeweils die im Zusammenhang mit dem ersten Ausführungsbeispiel beschriebene Struktur auf. Zum Verbinden der Elemente 12 und 14 bzw. 14 und 13 sind jeweils zwei gleichartige Verbindungsstücke 15 vorgesehen, die entsprechend den Verbindungsstücken 10 und 11 des ersten Ausführungsbeispiels ausgebildet sind. Die jeweiligen Außendurchmesser D4 bzw. D3 des des ersten bzw. des zweiten zylindermantelförmigen Abschnittes 15a, 15b sind an die jeweiligen Innendurchmesser der Elemente 12 und 14 angepaßt. Der Platzhalter gemäß dieses zweiten Ausführungsbeispiels wird hauptsächlich zum Verbinden von Wirbeln mit gleichem Querschnitt verwendet, wobei bei der Verbindung jedoch Platz gespart werden muß, so daß es vorteilhaft ist, das Mittelstück, in diesem Fall das Element 14 mit geringerem Durchmesser auszubilden.

Bei den oben beschriebenen Ausführungsbeispielen ist der Querschnitt der mantelförmigen Elemente jeweils kreisförmig ausgebildet. Bevorzugt wird jedoch der Querschnitt jeweils in Abhängigkeit von der Querschnittsform der zu verbindenden Wirbel verwendet. Für die Verbindung von Wirbeln im lumbalen Bereich weisen die mantelförmigen Elemente vorzugsweise einen nierenförmigen Querschnitt oder einen ovalen Querschnitt auf. Es besteht jedoch die Möglichkeit je nach Ausbildung der Abschnitte des Verbindungsstückes auch einen Platzhalter zu schaffen, der auf seinen beiden einander gegenüberliegenden Flächen, die jeweils mit den Wirbeln verbunden werden sollen, unterschiedliche Flächenformen vorzusehen.

Der Platzhalter muß nicht notwendigerweise aus drei mantelförmigen Elementen zusammengesetzt sein. In manchen Fällen genügen auch zwei Elemente oder es können mehr als drei Elemente erforderlich sein. Die geeignete Zusammensetzung des Platzhalters aus mehreren Elementen geeigneter Größe ermöglicht eine einfache Anpassung an die Gegebenheiten des Wirbelersatzes.

Anstelle des Ausfüllens mit Knochenzement ist es auch möglich, im Inneren des Platzhalters eigene oder Fremdknochenstücke einzusetzen, so daß der Platzhalter um das eingesetzte Knochenstück herum die Tragfunktion übernimmt. Damit wird ein sicherer Schutz des Rückenmarkkanales gegen Verschleudern des Knochenzementes oder der Knochenstücke erreicht.

## Patentansprüche

1. Platzhalter für Wirbel, gekennzeichnet durch wenigstens zwei mantelförmige Elemente (2, 3, 4, 12, 13, 14) mit unterschiedlichem Querschnitt dadurch gekennzeichnet, daß wenigstens ein Verbindungsstück (10, 11, 15) zum Verbinden der Elemente vorgesehen ist und das Verbindungsstück (10, 11, 15) als mantelförmiges Element ausgebildet ist mit einem ersten Abschnitt (10a, 11a, 15a), dessen äußerer Mantelquerschnitt dem inneren Mantelquerschnitt des einen mantelförmigen Elementes (2) entspricht und mit einem zweiten Abschnitt (10b, 11b), dessen äußerer Mantelquerschnitt dem inneren Mantelquerschnitt des mit dem ersten mantelförmigen Elementes zu verbindenden zweiten mantelförmigen Elementes (3) entspricht.

2. Platzhalter nach Anspruch 1, dadurch gekennzeichnet, daß
die Elemente (2, 3, 4, 12, 13, 14) eine zylindrische Form aufweisen.

3. Platzhalter nach Anspruch 1 oder 2, dadurch gekennzeichnet, daß
in der Wandung der mantelförmigen Elemente (2, 3, 4, 12, 13, 14) jeweils eine Ausnehmung (5) vorgesehen ist.

4. Platzhalter nach einem der Ansprüche 1 bis 3, dadurch gekennzeichnet, daß
mindestens einer der Ränder (8,9) eines jeden mantelförmigen Elementes (2, 3, 4, 12, 13, 14) wenigstens teilweise zackenförmig mit Zacken (8a, 8b, 9a, 9b) ausgebildet ist.

5. Platzhalter nach Anspruch 3 oder 4, dadurch gekennzeichnet, daß
die Wandung aus einem Blech gebildet ist, welches eine Vielzahl von annähernd viereckigen Ausnehmungen (5) aufweist, die so ausgerichtet sind, daß sich jeweils eine Diagonale der Ausnehmung im wesentlichen parallel zu einer Längsachse des Elementes (2, 3, 4, 12, 13, 14) erstreckt.

6. Platzhalter nach Anspruch 5, dadurch gekennzeichnet, daß
die Ausnehmungen (5) im wesentlichen rautenförmig sind und die Diagonale die Längsdiagonale der Raute ist.

7. Platzhalter nach Anspruch 5 oder 6, dadurch gekennzeichnet, daß
die Zacken (8a, 8b, 9a, 9b) jeweils durch V-förmige Ausnehmungen an den Rändern (8, 9) des Bleches gebildet sind.

8. Platzhalter nach einem der Ansprüche 4 bis 7, dadurch gekennzeichnet, daß
die Zacken (8a, 8b, 9a, 9b) zum besseren Eingreifen in benachbarte Wirbelteile angefast sind.

9. Platzhalter nach einem der Ansprüche 1 bis 8, dadurch gekennzeichnet, daß
jeder der beiden Abschnitte (10a, 10b; 11a, 11b; 15a, 15b) eine Gewindebohrung zur Aufnahme einer Schraube (50) aufweist.

10. Platzhalter nach einem der Ansprüche 1 bis 9, dadurch gekennzeichnet, daß
das Verbindungsstück (10, 11, 15) an der Verbindungsstelle der Abschnitte (10a, 10b; 11a, 11b) einen Vorsprung (20, 21) mit einem Querschnitt aufweist, der größer oder gleich dem größten Querschnitt der miteinander zu verbindenden Elemente (2, 3, 4, 12, 13, 14) ist.

11. Platzhalter nach einem der Ansprüche 1 bis 10, dadurch gekennzeichnet, daß
der erste und der zweite Abschnitt (10a, 10b; 11a, 11b) des Verbindungsstückes (10, 11) zylindermantelförmig sind.

12. Platzhalter nach einem der Ansprüche 1 bis 11, dadurch gekennzeichnet, daß
das Verbindungsstück mit den mantelförmigen Elementen verschraubt ist.

13. Platzhalter nach einem der Ansprüche 1 bis 12, dadurch gekennzeichnet, daß
der Platzhalter drei mantelförmige Elemente (2, 3, 4; 12, 13, 14) mit zwei zur Verbindung von jeweils zwei Elementen vorgesehene Verbindungsstücken (10, 11; 15, 15) aufweist.

14. Platzhalter nach Anspruch 13, dadurch gekennzeichnet, daß
zwei der drei mantelförmigen Elemente (12, 13) die über das dritte mantelförmige Element (14) miteinander verbunden sind, gleichen Querschnitt aufweisen.

15. Platzhalter nach einem der Ansprüche 1 oder 3 bis 14, dadurch gekennzeichnet, daß
die Elemente und die Verbindungsstücke einen nierenförmigen Querschnitt aufweisen.

16. Platzhalter nach einem der Ansprüche 1 bis 15, dadurch gekennzeichnet, daß
der Platzhalter aus einem körperverträglichen Material, insbesondere aus Titan, gebildet ist.

17. Platzhalter nach einem der Ansprüche 1 bis 16, dadurch gekennzeichnet, daß das innere der mantelförmigen Elemente mit einem Knochenzement ausgefüllt ist.

## Claims

1. Replacement for vertebrae, characterized by at least two envelope-shaped elements (2, 3, 4, 12, 13, 14) of different cross-section, characterized in that at least one connection piece (10, 11, 15) for connecting the elements is provided and the connection piece (10, 11, 15) is constructed as an envelope-shaped element with a first portion (10a, 11a, 15a) whose outer envelope cross-section corresponds to the inner envelope cross-section of the one envelope-shaped element (2) and with a second portion (10b, 11b) whose outer envelope cross-section corresponds to the inner envelope cross-section of the second envelope-shaped element (3), which is to be connected to the first envelope-shaped element.

2. Replacement according to Claim 1, characterized in that the elements (2, 3, 4, 12, 13, 14) have a cylindrical shape.

3. Replacement according to Claim 1 or 2, characterized in that an aperture (5) is provided in each case in the wall of the envelope-shaped elements (2, 3, 4, 12, 13, 14).

4. Replacement according to one of Claims 1 to 3, characterized in that at least one of the edges (8,9) of each envelope-shaped element (2, 3, 4, 12, 13, 14) is at least partly of toothed construction with teeth (8a, 8b, 9a, 9b).

5. Replacement according to Claim 3 or 4, characterized in that the wall is formed from a metal sheet having a large number of approximately quadrangular apertures (5) which are aligned such that in each case one diagonal of the aperture extends substantially parallel to a longitudinal axis of the element (2, 3, 4, 12, 13, 14).

6. Replacement according to Claim 5, characterized in that the apertures (5) are substantially rhombus-shaped and the diagonal is the longitudinal diagonal of the rhombus.

7. Replacement according to Claim 5 or 6, characterized in that the teeth (8a, 8b, 9a, 9b) are each formed by V-shaped cutouts at the edges (8, 9) of the metal sheet.

8. Replacement according to one of Claims 4 to 7, characterized in that the teeth (8a, 8b, 9a, 9b) are chamfered for better engagement in adjacent vertebra parts.

9. Replacement according to one of Claims 1 to 8, characterized in that each of the two portions (10a, 10b; 11a, 11b; 15a, 15b) has a threaded bore for receiving a screw (50).

10. Replacement according to one of Claims 1 to 9, characterized in that the connection piece (10, 11, 15) has, at the junction of the portions (10a, 10b; 11a, 11b), a projection (20, 21) with a cross-section which is greater than or equal to the greatest cross-section of the elements (2, 3, 4, 12, 13, 14) to be connected to one another.

11. Replacement according to one of Claims 1 to 10, characterized in that the first and the second portion (10a, 10b; 11a, 11b) of the connection piece (10, 11) are cylinder-envelope-shaped.

12. Replacement according to one of Claims 1 to 11, characterized in that the connection piece is screwed to the envelope-shaped elements.

13. Replacement according to one of Claims 1 to 12, characterized in that the replacement has three envelope-shaped elements (2, 3, 4; 12, 13, 14) with two connection pieces (10, 11; 15, 15) provided for connecting in each case two elements.

14. Replacement according to Claim 13, characterized in that two of the three envelope-shaped elements (12, 13) which are connected to each other by the third envelope-shaped element (14) have the same cross-section.

15. Replacement according to one of Claims 1 or 3 to 14, characterized in that the elements and the connection pieces have a kidney-shaped cross-section.

16. Replacement according to one of Claims 1 to 15, characterized in that the replacement is formed from a body-compatible material, in particular from titanium.

17. Replacement according to one of Claims 1 to 16, characterized in that the inner one [sic] of the envelope-shaped elements is filled with a bone cement.

## Revendications

1. Dispositif destiné à se mettre à la place d'une vertèbre, caractérisé par au moins deux éléments (2, 3, 4, 12, 13, 14) en forme d'enveloppe, et de section transversale différente, caractérisé en ce qu'au moins une pièce de liaison (10, 11, 15) est prévue pour assurer la liaison des éléments et en ce que la pièce de liaison (10, 11, 15) est réalisée comme éléments en forme d'enveloppe, avec un premier tronçon (10a, 11a, 15a) dont la section transversale extérieure d'enveloppe correspond à la section transversale intérieure d'enveloppe d'un premier élément (2) en forme d'enveloppe et avec un deuxième tronçon (10b, 11b) dont la section transversale extérieure d'enveloppe correspond à la section transversale intérieure d'enveloppe du deuxième élément (3) en forme d'enveloppe assurant la liaison au premier élément en forme d'enveloppe.

2. Dispositif destiné à se mettre à la place d'une vertèbre selon la revendication 1, caractérisé en ce que les éléments (2, 3, 4, 12, 13, 14) présentent une forme cylindrique.

3. Dispositif destiné à se mettre à la place d'une vertèbre selon la revendication 1 ou la revendication 2, caractérisé en ce qu'un évidement (5) est prévu dans la paroi de chacun des éléments (2, 3, 4, 12, 13, 14) en forme d'enveloppe.

4. Dispositif destiné à se mettre à la place d'une vertèbre selon l'une des revendications 1 à 3, caractérisé en ce qu'au moins l'un des bords (8, 9) de chaque élément (2, 3, 4, 12, 13, 14) en forme d'enveloppe est réalisé au moins partiellement en forme de pointe, avec des pointes (8a, 8b, 9a, 9b).

5. Dispositif destiné à se mettre à la place d'une vertèbre selon la revendication 3 ou la revendication 4, caractérisé en ce que la paroi est réalisée à partir d'une tôle qui présente une pluralité d'évidements (5) approximativement quadrangulaires, orientés de manière à ce que pour chacun une diagonale de l'évidement s'étende de façon sensiblement parallèle à un axe longitudinal de l'élément (2, 3, 4, 12, 13, 14).

6. Dispositif destiné à se mettre à la place d'une vertèbre selon la revendication 5, caractérisé en ce que les évidements (5) ont sensiblement la forme d'un losange et en ce que la diagonale est la diagonale longitudinale du losange.

7. Dispositif destiné à se mettre à la place d'une vertèbre selon la revendication 5 ou la revendication 6, caractérisé en ce que les pointes (8a, 8b, 9a, 9b) sont chacune constituées par des évidements en forme de V ménagés sur les bords (9, 9) de la tôle.

8. Dispositif destiné à se mettre à la place d'une vertèbre selon l'une des revendications 4 à 7, caractérisé en ce que les pointes (8a, 8b, 9a, 9b) sont chanfreinées pour assurer un meilleur engagement dans des parties de vertèbres voisines.

9. Dispositif destiné à se mettre à la place d'une vertèbre selon l'une des revendications 1 à 8, caractérisé en ce que chacun des deux tronçons (10a, 10b; 11a, 11b; 15a, 15b) présente un trou taraudé destiné à recevoir une vis (50).

10. Dispositif destiné à se mettre à la place d'une vertèbre selon l'une des revendications 1 à 9, caractérisé en ce que la pièce de liaison (10, 11, 15) présente, au point de liaison des tronçons (10a, 10b; 11a, 11b), une saillie (20, 21) ayant une section transversale plus grande que, ou égale à, la plus grande section transversale des éléments (2, 3, 4, 12, 13, 14) à relier ensemble.

11. Dispositif destiné à se mettre à la place d'une vertèbre selon l'une des revendications 1 à 10, caractérisé en ce que le premier et le deuxième tronçons (10a, 10; 11a, 11b) de la pièce de liaison (10, 11) sont réalisés en forme d'enveloppe cylindrique.

12. Dispositif destiné à se mettre à la place d'une vertèbre selon l'une des revendications 1 à 11, caractérisé en ce que la pièce de liaison est vissée aux éléments en forme d'enveloppe.

13. Dispositif destiné à se mettre à la place d'une vertèbre selon l'une des revendications 1 à 12, caractérisé en ce que le dispositif pour garder un espace comporte trois éléments en forme d'enveloppe (2, 3, 4; 12, 13, 14) ayant deux pièces de liaison (10, 11; 15, 15) prévues pour assurer la liaison chaque fois de deux éléments.

14. Dispositif destiné à se mettre à la place d'une vertèbre selon la revendication 13, caractérisé en ce que deux (12, 13) des trois éléments en forme d'enveloppe, qui sont reliés ensemble par l'intermédiaire du troisième élément en forme d'enveloppe (14), ont les mêmes sections transversales.

15. Dispositif destiné à se mettre à la place d'une vertèbre selon l'une des revendications 1 ou 3 à 14, caractérisé en ce que les éléments et les pièces de liaison présentent une section transversale de forme oblongue.

16. Dispositif destiné à se mettre à la place d'une vertèbre selon l'une des revendications 1 à 15, caractérisé en ce que le dispositif destiné à se mettre à la place est constitué d'un matériau biocompatibles, en particulier de titane.

17. Dispositif destiné à se mettre à la place d'une vertèbre selon l'une des revendications 1 à 16, caractérisé en ce que l'intérieur des éléments en forme d'enveloppe est rempli d'un ciment pour os ou ostéotrope.
